# FASCICULE DE BREVET EUROPEEN

(11) **EP 2 349 214 B1**
(45) Date de publication et mention de la délivrance du brevet: **11.04.2018**
(21) Numéro de dépôt: 09736245.3
(22) Date de dépôt: 06.08.2009
(51) Int. Cl.: A61K 9/16, A61K 9/50

(54) **COMPOSITION PHARMACEUTIQUE SOUS FORME DE GRANULES ET PROCEDE DE FABRICATION D'UNE TELLE COMPOSITION PHARMACEUTIQUE**
PHARMAZEUTISCHE ZUSAMMENSETZUNG IN GRANULATFORM UND VERFAHREN ZUR HERSTELLUNG EINER SOLCHEN PHARMAZEUTISCHEN ZUSAMMENSETZUNG
PHARMACEUTICAL COMPOSITION IN GRANULE FORM AND METHOD FOR THE PRODUCTION OF SUCH A PHARMACEUTICAL COMPOSITION

(30) Priorité: 06.08.2008 FR 0804496
(43) Date de publication de la demande: 03.08.2011
(73) Titulaire: Laboratoires Docteur Gaetano Zannini, 83700 Saint Raphaël (FR)
(72) Inventeur: MATHONNET, Jean-Pierre, F-06110 Le Cannet (FR); ZANNINI, Gaetano, F-06410 Biot (FR)
(74) Mandataire: Macquet, Christophe
(86) Numéro de dépôt international: PCT/FR2009/000987
(87) Numéro de publication internationale: WO 2010/015754

(56) Documents cités:
- EP-A- 0 222 914
- EP-A- 0 253 684
- WO-A-02/40144
- WO-A-97/04861
- WO-A1-2007/058645
- FR-A- 2 737 120

## Description

L'invention concerne une composition comprenant au moins un composé pharmaceutiquement actif ainsi qu'un procédé de fabrication d'une telle composition pharmaceutique.

Dans le domaine des compositions pharmaceutiques solides absorbées par voie orale, on utilise couramment des formulations à dissoudre dans un liquide, à avaler, à sucer, à croquer, sublinguales ou encore orodispersibles. Les formulations connues de l'art antérieur se présentent en particulier sous la forme de comprimés, dragées, ou encore lyoc.

Les comprimés peuvent être effervescents, à dissoudre dans un liquide. Toutefois, ceux-ci peuvent avoir un mauvais goût et ne sont pas adaptés au traitement ambulatoire en l'absence d'eau.

On connaît également les comprimés à avaler. Toutefois, pour certains patients, notamment les personnes âgées ou les jeunes enfants, ces comprimés sont désagréables, difficiles voire impossibles à avaler, même avec de l'eau. Cela peut avoir comme conséquence la non-observance thérapeutique et l'absence d'efficacité du traitement. De plus, ces comprimés laissent souvent un mauvais goût dans la bouche.

Aussi, les comprimés à avaler peuvent être enrobés, on parle alors de dragées. L'enrobage présente quelquefois une action thérapeutique mais il sert généralement à masquer un mauvais goût ou une odeur désagréable, ou à protéger le principe actif des sucs gastriques et intestinaux.

Les comprimés peuvent également se présenter sous la forme de comprimés à sucer ou de comprimés sublinguaux lorsque le principe actif peut passer dans le sang via les muqueuses de la bouche, en particulier les muqueuses du dessous de la langue richement vascularisée.

Dans l'art antérieur, on trouve également des formulations orodispersibles comprenant un ingrédient pharmaceutiquement actif. Elles se désagrègent en quelques secondes dans la bouche, permettant de prendre de gros comprimés sans risque de fausse route. Simples d'utilisation, elles s'avèrent parfaitement adaptées au traitement ambulatoire.

Il existe plusieurs technologies pour obtenir une formulation orodispersible à délitement buccal rapide. Par exemple, la formulation de type lyoc est préparée à partir d'une masse pâteuse qui est coulée dans des alvéoles puis introduite dans le lyophilisateur. Après dessiccation, les lyocs se présentent en unités de la taille d'un comprimé. La formulation lyoc permet une prise immédiate et facilitée de comprimés en absence d'eau. Toutefois, la sensation en bouche de cette masse pâteuse est désagréable, même en présence d'arôme.

Il existe également la technologie Flashtab™ d'Ethypharm qui offre un comprimé aromatisé qui fond rapidement dans la bouche, sans eau et qui présente plusieurs avantages en termes d'acceptabilité, de précision dans les dosages et de sécurité. Cette technologie est utilisée pour améliorer l'administration et le goût des médicaments difficiles à avaler et de mauvais goût. Les comprimés sont composés de microparticules qui se dispersent rapidement dans la bouche, permettant ainsi la libération rapide des molécules actives dans l'organisme. Ils peuvent être pris n'importe où sans eau et n'ont pas besoin d'être mâchés. Une fois avalées, les particules libèrent la substance active dans la partie basse du tractus gastro-intestinal.

L'ensemble des formulations précitées doivent être adaptées de par leur procédé de fabrication pour pouvoir comprendre plusieurs substances actives. Des tentatives pour fabriquer de tels comprimés ont déjà été réalisées, en comprimant par exemple un mélange unique comprenant à la fois des excipients de compression et des substances actives. Cependant les comprimés obtenus présentent certains inconvénients, notamment une répartition hétérogène de chacune des substances actives au sein d'un comprimé et d'un comprimé à un autre, ou un risque d'incompatibilité entre les différents composants du comprimé, substances actives ou excipients.

Les mélanges de poudre utilisés pour la fabrication de tels comprimés comprenant plusieurs actifs sont généralement complexes à maîtriser car ils sont constitués de plusieurs populations de particules de substances actives et d'excipients, chacune ayant ses propres caractéristiques de taille, de densité et/ou de formes. De cette hétérogénéité découle un risque accru de ségrégation, qui se traduit par un démélange progressif de certaines populations de particules, au cours du stockage ou dans la trémie d'alimentation de la machine à comprimer. La forme unitaire finale présente alors une teneur hautement variable en chacune des substances actives, et des caractéristiques intrinsèques de dureté, de désintégration ou de palatabilité significativement différentes dans un même lot.

Ainsi, une première difficulté technique est d'obtenir une répartition homogène de chaque substance active au sein de chaque comprimé ainsi que des teneurs homogènes d'un comprimé à un autre.

Une deuxième difficulté est de conserver des teneurs constantes de chaque substance active, tout au long du procédé de fabrication des comprimés.

De plus, une troisième difficulté technique pour réaliser des comprimés comprenant une association de principes actifs est le choix des substances actives et des excipients pouvant être mis en oeuvre ensemble, en raison d'un risque d'incompatibilité entre les substances actives elles-mêmes ou entre une substance active et des excipients. Ce risque augmente avec le nombre de composants présents dans le comprimé.

Afin de réduire ces risques d'incompatibilité, des comprimés multicouches ont été proposés, formés d'au moins deux couches adhérant entre elles par une surface. Les couches du comprimé possèdent chacune leur propre composition et sont successivement formées par un cycle de compression. Cela limite à la fois les risques d'hétérogénéité de teneur et d'incompatibilité physico-chimique des principes actifs et des excipients. La cohésion des différentes couches est assurée par l'application de forces de compression élevées aboutissant à des comprimés ayant des valeurs de dureté souvent bien supérieures à 100 N, ou par la présence d'un liant dans au moins une des couches du comprimé, en quantité efficace pour promouvoir l'adhésion entre les couches. La préparation d'un comprimé multicouche nécessite de répéter l'application de forces de compression sur chaque mélange de poudre.

Ces conditions ne sont donc favorables ni dans le cas de comprimés destinés à se désintégrer rapidement, l'effet de la compression entrainant une désagrégation lente, ni dans le cas de substances actives nécessitant un masquage préalable de leur goût. En effet, les moyens de masquage du goût, tels que l'enrobage polymérique, sont particulièrement sensibles à la compression. L'application de forces de compression élevées accroît notamment le risque de fissuration et de rupture du film polymérique, ce qui conduit à la perte partielle ou totale des propriétés du film. Une telle fissuration altère irréversiblement le profil de libération du ou des principes actifs qu'ils contiennent, la protection du principe actif vis à vis de l'environnement et le masquage du goût du principe actif.

On connaît également des consommables à mâcher dans lesquels la ou les substances actives sont uniquement contenues dans l'enrobage du consommable (WO 2007/058645). Dans ce cas, il existe une différence d'absorption notable entre un tel consommable et un produit à sucer.

C'est pourquoi, à ce jour, parmi les formes solides destinées à se désintégrer dans la bouche, il n'existe que des comprimés multicouches sous forme de pastilles à sucer et destinés uniquement à l'administration de substances actives à action locale limitée à la muqueuse buccale et à l'oropharynx.

De plus, ces comprimés ne comprennent que des substances actives qui ne nécessitent pas de masquage de goût autrement que par la simple addition d'édulcorants.

Ainsi, les formulations pharmaceutiques de l'art antérieur et leurs procédés de préparation présentent de multiples inconvénients.

Compte tenu de ce qui précède, un problème que se propose de résoudre l'invention est de réaliser une composition ayant une concentration en composé pharmaceutiquement actif supérieure ou égale à 10% en poids du poids total de la composition qui ne présente pas les inconvénients liés aux formulations selon l'art antérieur et en particulier supprimer les pics de mauvais goût liés à la libération des actifs tout en présentant une forte concentration en composé actif.

La solution de l'invention à ce problème posé a pour premier objet une composition orosoluble comprenant au moins un composé pharmaceutiquement actif présent, dans ladite composition, à une concentration supérieure ou égale à 10% en poids du poids total de la composition, caractérisée en ce qu'elle se présente sous la forme de granules comprenant :
un noyau obtenu par granulation en présence d'une phase solide poudreuse cristallisée et d'une phase liquide ; et
plusieurs enrobages successifs obtenus par pulvérisation d'une phase liquide et saupoudrage d'une phase solide poudreuse cristallisée,
le composé pharmaceutiquement actif étant présent dans ledit noyau et/ou dans les enrobages successifs.

De plus, elle a pour second objet, un procédé de fabrication d'une composition selon l'invention, caractérisé en ce qu'il comprend les étapes suivantes de:
granulation en présence d'une phase solide poudreuse cristallisée et d'une phase liquide comprenant notamment une gomme ;
plusieurs enrobages successifs par pulvérisation d'une phase liquide et saupoudrage d'une phase solide poudreuse cristallisée, au moins un composé pharmaceutiquement actif étant présent dans la phase solide poudreuse et/ou dans la phase liquide, à l'étape de granulation et/ou dans d'enrobage ;
   - séchage à l'issue de chaque étape d'enrobage des granules enrobés obtenus ; et
   - tamisage en vue de l'obtention de granules calibrés.

Ainsi, la composition selon l'invention présente notamment les avantages suivants :
- une texture qui procure au patient une sensation en bouche plus agréable ;
- un bon goût ;
- une meilleure observance du traitement par le patient ;
- une formulation préférée en granules orosolubles qui facilite l'ingestion par le patient;
- éventuellement l'association compatible de deux ou plusieurs substances actives dans la même composition ;
- des concentrations homogènes et contrôlées pour chacun des composés actifs au sein du noyau et/ou de chaque enrobage ainsi que d'une composition à l'autre ;
- une répartition constante du composé actif au sein de chaque enrobage ;
- une conservation stable de la composition obtenue ;
- une coque externe intacte de par l'absence de force de compression ;
- une coque externe ne comprenant pas d'édulcorant ; et
- une protection du ou des principes actifs vis à vis de l'environnement, notamment pour les principes actifs particulièrement sensibles à l'air.

De manière avantageuse : - la concentration en composé pharmaceutiquement actif est comprise entre 20 et 60 % en poids du poids total de la composition ; - le composé pharmaceutiquement actif est présent dans les enrobages successifs et la concentration en composé pharmaceutiquement actif est homogène dans lesdits différents enrobages ; - le composé pharmaceutiquement actif est présent dans le noyau et la concentration en composé pharmaceutiquement actif est homogène dans le noyau et dans les enrobages successifs, soit du centre à la périphérie de la composition ; - la phase solide et la phase liquide pulvérisées dans l'étape d'enrobage sont différentes de celles utilisées dans l'étape de granulation, et/ou sont différentes d'une étape d'enrobage à une autre ; - le noyau comporte une gomme ; - le composé pharmaceutiquement actif est présent dans les enrobages successifs et la répartition du composé actif est sensiblement constante dans chacun des enrobages ; - le composé pharmaceutiquement actif est choisi parmi le paracétamol, l'acide acétylsalicilique, l'ibuprofène, la dompéridone, la lopéramide le dextrométhorphane, la codéine, la carbocystéine, l'acétylcystéine, la N-acétylcystéine, l'acide hyaluronique, la guaifénésine, la tyrothricine, le chlorure de cétylpyridinum, l'hexétidine, pris seul ou en combinaison ; - le composé pharmaceutiquement actif est incorporé sous forme de poudre dans la phase solide poudreuse et/ou sous forme d'extrait liquide dans la phase liquide à l'étape de granulation et/ou d'enrobage ; - le procédé comprend une étape supplémentaire de réalisation d'une coque externe par pulvérisation d'une phase liquide comprenant un composé aromatique sur le granule préalablement formé ; - la composition pour son utilisation comme médicament ; - la composition pour le traitement de la douleur, de la fièvre, des nausées, des vomissements, des maux de voyage, des diarrhées, de la toux, des maux de gorge, des maux de ventre, des brûlures d'estomac. Cette liste n'est pas exhaustive.

L'invention sera mieux comprise à la lecture de la description non limitative qui va suivre.

Dans l'ensemble du présent texte, à moins qu'il ne soit spécifié autrement, il est entendu que, lorsque des intervalles de concentrations sont donnés, ils incluent les bornes supérieure et inférieure dudit intervalle. De même, sauf indication contraire, les proportions des différents constituants de la composition sont exprimées en pourcentage en poids du poids total de ladite composition.

Selon l'invention, la composition comprend au moins un composé pharmaceutiquement actif présent, dans ladite composition, à une concentration supérieure ou égale à 10% en poids du poids total de la composition.

Selon l'invention, le procédé de fabrication de la composition comprend les étapes suivantes de - granulation en présence d'une phase solide poudreuse cristallisée et d'une phase liquide comprenant notamment une gomme ; - plusieurs enrobages successifs par pulvérisation d'une phase liquide et saupoudrage d'une phase solide poudreuse cristallisée, au moins un composé pharmaceutiquement actif étant présent dans la phase solide poudreuse et/ou dans la phase liquide, à l'étape de granulation et/ou d'enrobage ; - séchage à l'issue de chaque étape d'enrobage des granules enrobés obtenue ; et - tamisage en vue de l'obtention de granules calibrés.

On retrouve avantageusement les étapes du procédé de granulation dit Tecnabio™ divulgué dans le document brevet publié sous le numéro WO 97/04861, qui met en oeuvre un appareillage pouvant être notamment une turbine rotative du type turbine à dragéification en acier inoxydable.

Pour la fabrication selon l'invention, on utilise encore plus avantageusement les étapes du procédé de granulation dit Tecnabio™ II qui comporte une étape de séchage dynamique.

La composition selon l'invention se présente sous la forme de granules comprenant un noyau obtenu par granulation en présence d'une phase solide poudreuse cristallisée et d'une phase liquide et plusieurs enrobages successifs obtenus par pulvérisation d'une phase liquide et saupoudrage d'une phase solide poudreuse cristallisée, le composé pharmaceutiquement actif étant présent dans ledit noyau et/ou dans les enrobages successifs.

Dans la composition selon l'invention, le composé pharmaceutiquement actif est présent dans les enrobages successifs. La concentration en composé pharmaceutiquement actif est, de préférence, homogène dans les différents enrobages. De plus, la répartition du composé actif est sensiblement constante dans chacun des enrobages.

Le composé pharmaceutiquement actif est également présent dans le noyau de la composition selon l'invention. La concentration en composé pharmaceutiquement actif est homogène dans le noyau et dans les enrobages successifs, soit du centre à la périphérie de la composition.

Par homogène, on entend notamment des granules ayant une constitution identique les uns par rapport aux autres et une uniformité de la concentration en composé pharmaceutiquement actif du centre à la périphérie du granule.

Selon un autre mode de réalisation, la composition peut être différente entre le noyau et la(les) couche(s) d'enrobage(s) et/ou d'une couche d'enrobage à une autre. Ainsi, le noyau peut avoir une certaine composition, les couches suivantes une composition différente. Le granule peut également comprendre une coque externe, qui peut avoir une composition encore différente. Enfin, une coque encore plus externe peut encore être réalisée pour isoler encore l'ensemble du milieu extérieur.

Dans une première étape du procédé selon l'invention, on effectue une granulation en présence d'une phase solide poudreuse formée par une substance cristallisée inerte et d'une phase liquide comprenant au moins une gomme, de l'eau et de l'alcool.

La substance cristallisée inerte peut comprendre/être choisie parmi le talc, ou toutes autres substances cristallisées inertes choisies dans le groupe comprenant le kaolin, la silice, l'alumine, le carbonate de calcium, les sucres, les polyols et les maltodextrines, ou un mélange de ceux-ci. Cette liste n'est pas exhaustive. Les composants de la phase liquide peuvent être ajoutés les uns après les autres.

La gomme peut être choisie parmi les gommes hydrosolubles telles que la gomme arabique, les carraghénanes, les alginates, les pectines, la gomme karaya, la gomme xanthane, le chitosan ou ses dérivés, l'acide hyaluronique, les polymères pullulan ou agar, le dextran, les celluloses et leurs dérivés tels que la méthylcellulose ou l'hydroxypropylcellulose, les polymères de l'acide acrylique réticulés avec un éther allylique, tels que ceux connus sous le nom générique de carbomers, essentiellement constitués d'un carbomer ou d'un mélange de carbomers tels que les polymères de l'acide acrylique réticulés avec des polyalkenyl éthers sous la dénomination Carbopol™.

L'alcool peut être choisi par exemple parmi l'éthanol, l'isopropanol.

Sur le plan quantitatif, par exemple, pour 100 g de phase solide poudreuse utilisés pour la granulation, on utilise entre 5 et 15 g de gomme, entre 25 et 45 g d'eau et entre 25 et 45 g d'alcool. La granulation est effectuée sensiblement à température ambiante et en présence d'une hygrométrie inférieure à 70%.

Dans une deuxième étape du procédé de fabrication de la composition selon l'invention, on effectue plusieurs enrobages successifs du noyau obtenu dans la première étape. Ces enrobages sont réalisés par pulvérisation d'une phase liquide précitée puis saupoudrage d'une phase solide poudreuse cristallisée.

Pour chacune des compositions selon l'invention, un composé pharmaceutiquement actif peut être apporté dans une phase solide poudreuse, de préférence sous forme d'extraits secs, et/ou dans une phase liquide, de préférence sous forme d'extraits liquides.

L'enrobage se fait par un processus dynamique permettant l'obtention d'une concentration homogène du composé pharmaceutiquement actif du centre à la périphérie du granule. Le diamètre du granule augmente progressivement jusqu'à l'obtention du rapport diamètre / dose désiré, et en conséquence le dosage correct en composé pharmaceutiquement actif du produit final sous forme de granules.

A titre d'exemple, le dépôt d'une couche d'enrobage par imprégnation, c'est-à-dire par pulvérisation d'une solution collante liquide sur le support solide, par exemple sur le noyau, et en effectuant une pause permettant l'imprégnation du noyau, avant le dépôt d'une poudre micronisée, prend un temps de 5 minutes.

Les couches périphériques d'enrobage sont ainsi obtenues par granulation d'une phase liquide, de poudres cristallines et d'au moins un composé pharmaceutiquement actif hydrosoluble ou non.

Le procédé permet donc d'intégrer dans le noyau et/ou les couches d'enrobage successives de la composition selon l'invention tout type de composé pharmaceutiquement actif quelque soit leur solubilité dans l'eau ou les solvants organiques.

Les deux premières étapes du procédé selon l'invention sont avantageusement réalisées à une température comprise entre 18 et 25°C.

Selon un mode de réalisation, la phase liquide et la phase solide pulvérisée dans l'étape d'enrobage sont identiques de celles utilisées dans l'étape de granulation. Le granule obtenu comporte alors plusieurs couches mais sa constitution est homogène de son coeur à sa périphérie. En d'autres termes, la composition du noyau et de chaque couche est identique. En particulier, la concentration en composé pharmaceutiquement actif est homogène du centre à la périphérie de chaque granule et la répartition est constante au sein de chaque enrobage.

Selon un autre mode de réalisation, la phase liquide et la phase solide pulvérisées dans l'étape d'enrobage sont différentes de celles utilisées dans l'étape de granulation. Elles peuvent également être identiques ou bien varier au fur et à mesure de l'étape d'enrobage. On obtient alors des granules avec une constitution différente entre le noyau et la(les) couche(s) d'enrobage(s) et/ou d'une couche d'enrobage à une autre.

Le granule peut être de toute forme et de toute taille. Préférentiellement, le granule est sensiblement sphérique de diamètre moyen supérieur à environ 2 mm, de préférence compris entre environ 5 mm et environ 20 mm.

La composition des granules est identique d'un granule à l'autre.

A titre d'exemple non limitatif, le composé pharmaceutiquement actif est choisi parmi le paracétamol, l'acide acétylsalicilique, l'ibuprofène, la dompéridone, la lopéramide le dextrométhorphane, la codéine, la carbocystéine, l'acétylcystéine, la N-acétylcystéine, l'acide hyaluronique, la guaifénésine, la tyrothricine, le chlorure de cétylpyridinum, l'hexétidine, pris seul ou en combinaison. Cette liste n'est pas exhaustive.

Selon l'invention, la concentration en composé pharmaceutiquement actif est supérieure ou égale à 10% en poids du poids total de la composition, préférentiellement comprise entre 20% et 60% en poids du poids total de composition sous forme de granule. Encore plus préférentiellement, la concentration est choisie de sorte à obtenir un granule ayant un dosage en composé pharmaceutiquement actif adapté au traitement. En particulier, les composés pharmaceutiquement actifs choisis parmi l'acétylcystéine, la N-acétylcystéine et l'acide hyaluronique sont compris entre 25% et 55%, de préférence 30% et 45% en poids du poids total de la composition.

La composition sous forme de granule comprend en outre entre 2% et 50% et, préférentiellement, entre 5% et 20% en poids de gommes notamment végétales et/ou synthétiques du poids total de composition sous forme de granule.

Le granule comprend en outre 20% à 80% d'une substance cristallisée inerte telle définie précédemment, en poids du poids total de composition sous forme de granule.

Il comprend enfin une quantité résiduelle d'eau, par exemple comprise entre environ 1% et environ 6% en poids du poids total de composition sous forme de granule, et une quantité résiduelle d'alcool. Ces quantités résiduelles sont éliminées lors du séchage.

Selon un autre mode de réalisation avantageux, la composition selon l'invention est susceptible de comprendre une pluralité de composés pharmaceutiquement actifs qui peuvent respectivement être apportés de manière compatible dans des couches concentriques indépendantes au cours de la granulation et/ou de l'enrobage. Dans ce cas en particulier, la composition du granule, notamment en composé pharmaceutiquement actif, peut être différente entre le noyau et la(les) couche(s) d'enrobage(s) et/ou d'une couche d'enrobage à une autre.

L'apport d'une pluralité d'actifs dans des couches concentriques différentes permet une cohabitation compatible au sein du granule en évitant toute interaction médicamenteuse non souhaitée. Ceci permet la libération de chaque actif de façon séparée et distincte évitant ainsi toute interaction incompatible au cours de la prise de la composition selon l'invention sous forme de granule.

Une synergie active peut éventuellement s'établir si l'on souhaite une interaction entre les deux actifs au moment de leur libération. On incorporera de tels actifs dans des couches concentriques distinctes mais proches de sorte à permettre leur interaction lors de la prise du granule.

Dans une troisième étape, un séchage en étuve ou dynamique des granules est effectué à l'issue de chaque étape d'enrobage.

Dans le cas du séchage en étuve, la température de séchage est avantageusement comprise entre environ 40°C et environ 55°C et, préférentiellement, entre environ 45°C et environ 50°C. Le temps de séchage est supérieur à 30 min, par exemple de l'ordre d'1h30.

Selon un mode de réalisation avantageux du procédé selon l'invention, le séchage peut être dynamique. Le séchage comprend alors plusieurs étapes différenciées notamment suivant la température du flux gazeux qui est projeté sur les granules.

Le séchage dynamique est réalisé dans une turbine qui est la même que la turbine ayant servi à enrober le noyau. Par ailleurs, on maintient la turbine en rotation de façon à poursuivre le mouvement des granules. Cela optimise fortement la circulation du flux gazeux servant au séchage.

Dans un premier temps, un séchage dit doux est réalisé par projection d'un flux gazeux à une température comprise entre 25°C et 35°C. Cette étape de séchage dit doux permet un séchage à coeur et donc en profondeur du granule enrobé et assure une montée progressive en température du granule.

Dans un deuxième temps, un séchage plus fort est réalisé par projection d'un flux gazeux à une température comprise entre 40°C et 50°C. Cette étape à température plus élevée accélère la vitesse de séchage du granule. En particulier, la dernière couche déposée sera plus rapidement fixée suivant cette étape.

Dans un troisième temps, un séchage terminal est réalisé en projetant un flux gazeux à nouveau à une température comprise entre 25°C et 35°C. Pour des raisons de simplicité, on pourra utiliser un flux à même température que celui utilisé pour le séchage dit doux préliminaire. Le séchage terminal permet de redescendre progressivement la température des granules avant, préférentiellement, une étape de refroidissement jusqu'à la température ambiante.

Concernant les temps de séchage, l'exemple suivant illustre la rapidité du cycle, pour une fabrication de 5 kg de granules dans une turbine d'essais :
- séchage dit doux : 5 minutes à 30°C
- séchage plus fort : 10 minutes à 45°C
- séchage terminal : 5 minutes à 30°C.

L'étape de refroidissement peut être réalisée sous un flux d'air à température ambiante, c'est-à-dire sensiblement autour de 20°C. A titre d'exemple, un refroidissement à température ambiante de 5 minutes est opéré.

De façon préférée, les flux gazeux projetés sur les granules sont des flux d'air purifiés et filtrés.

Lorsque le refroidissement est obtenu, on peut renouveler les phases d'enrobage et de séchage autant que nécessaire.

Pour l'obtention de granules calibrés, on effectue dans une quatrième étape, un tamisage. Les mailles des tamis sont adaptées pour l'obtention de granules de calibre désiré.

Le tamisage des granules n'est pas nécessaire après chaque enrobage. Seuls quelques tamisages, avantageusement un seul tamisage en fin d'opération est effectué.

La répétition des phases d'enrobage, de séchage, de tamisage permet d'obtenir des granules calibrés de dimension souhaitée.

A titre d'exemple, on effectue un tamisage avec des mailles à partir de 2 mm de diamètre jusqu'au diamètre souhaité. On obtient par exemple en fin de granulation des granules calibrés de 200 à 500 mg de poids unitaire moyen.

Aussi, à titre d'exemple, la fabrication d'un granule enrobé par une couche prend environ 1h40, avantageusement 15 min avec un chauffage dynamique.

Les exemples ci-dessous concernent la réalisation de compositions selon l'invention.

Selon un premier mode de réalisation, la composition des granules est sensiblement de

| | |
|---|---|
| N-Acétyl cystéine : | 35% |
| Arôme : | 3% |
| Maltitol : | QSP |

Selon un second mode de réalisation, la composition des granules est sensiblement de

| | |
|---|---|
| Ibuprofène : | 44% |
| Arôme : | 2% |
| Maltisorb : | QSP |

Selon un mode particulier de réalisation de l'invention, le procédé comporte une étape supplémentaire de fabrication d'une coque externe. Cette coque permet de protéger les principes actifs du milieu extérieur, notamment les principes actifs particulièrement sensibles à l'air.

Pour la fabrication de cette coque, on prépare tout d'abord une phase liquide comprenant une solution hydro-alcoolique de gomme, au moins un composé aromatique. Sur le plan quantitatif, cette phase liquide comporte entre 30% et 50% en poids de gomme, entre 1% et 20% en poids de composé aromatique et entre 5% et 20% en poids d'alcool (évaporé lors du séchage), les pourcentages étant donnés en poids du poids total de la phase considérée.

A titre d'exemple non limitatif, les gommes peuvent être choisies parmi les gommes naturelles.

A titre d'exemple non limitatif, le composé aromatique est choisi parmi les huiles essentielles, les essences.

On pulvérise alors cette phase liquide sur les granules préalablement réalisés. Cette phase assure notamment l'adhésion de la coque au granule. Puis, on saupoudre le tout d'une phase poudreuse cristallisée inerte. On effectue ensuite un dernier séchage en vue d'obtenir les granules selon l'invention.

Selon un mode de réalisation, la composition selon l'invention est orosoluble et destinée à être croquée, sucée ou à se dissoudre dans la bouche, préférentiellement sous la langue.

Par composition orosoluble, on entend une composition soluble dans son intégralité dont la solubilité, en particulier dans la cavité buccale, est fonction de la qualité et de la quantité du ou des composés pharmaceutiquement actifs et des excipients formant la composition.

Avantageusement, la composition orosoluble se dissout progressivement de manière intrinsèque au cours du temps ou par succion, en libérant immédiatement et progressivement le composé pharmaceutiquement actif présent dans les différentes couches concentriques du granule. Par exemple, les composés pharmaceutiquement actifs incorporés dans des mêmes couches au cours de la fabrication de la composition selon l'invention sont libérés simultanément et peuvent éventuellement interagir lors de leur libération. Avantageusement également, les composés pharmaceutiquement actifs incorporés dans des couches indépendantes et distinctes ont une action différée par libération progressive et séparée dans le temps.

Par exemple, la composition orosoluble selon l'invention se présente sous la forme d'un granule à sucer qu'on garde sous la langue ou contre la joue. Le temps de contact avec la muqueuse buccale est de plusieurs minutes, durant lesquelles la pénétration du composé pharmaceutiquement actif libéré se fait dès les capillaires sanguins de la muqueuse buccale, sublinguale. Avanatageusement, une telle composition orosoluble offre un mode d'administration qui évite les interactions entre les composés actifs, absorbés essentiellement au niveau gastrique, duodénal ou intestinal lors d'une prise orale, avec les sucs digestifs ainsi qu'avec le cycle entéro-hépatique en premier passage. De plus, une telle composition orosoluble présente l'avantage d'offrir une action plus intense et plus rapide que lors d'une prise orale, le temps de délitement du granule prolongé dans la bouche prolongeant la durée d'action du composé pharmaceutiquement actif ainsi libéré.

A titre d'exemple, lorsque la composition sous forme de granule est sensiblement sphérique, cette forme est conservée jusqu'à la dissolution complète du granule.

La composition sous forme de granules selon l'invention offre une agrégation synergique qui permet une bio disponibilité unique pour plus d'efficacité et de rapidité.

Lorsqu'ils se présentent sous la forme de granules à sucer, les granules selon l'invention ont une dureté élevée, comprise entre 100 et 500 N permettant un temps de séjour dans la cavité buccale de plusieurs minutes, correspondant au temps pendant lequel le granule se désintègre progressivement.

Lorsqu'ils se présentent sous la forme de granules sublinguaux, les granules selon l'invention ont une dureté moins élevée, comprise entre 10 et 100 N, préférentiellement entre 15 et 50 N.

L'érosion et la solubilisation, principaux mécanismes de désintégration du granule, dépendent alors directement de la taille du granule et de sa surface de contact avec la salive.

Avantageusement, les compositions orosolubles solides sont à sucer, ou encore sublinguales afin que les composés actifs puissent se dissoudre directement dans la bouche, préférentiellement sous la langue, et agir immédiatement en évitant les enzymes de la digestion et le cycle entéro-hépatique.

Selon un autre mode de réalisation, la composition selon l'invention est effervescente, à dissoudre dans un liquide.

La composition est utilisée comme médicament.

La composition est plus particulièrement utilisée pour le traitement de la douleur, la fièvre, des nausées, des vomissements, des maux de voyage, des diarrhées, de la toux, des maux de gorge, des maux de ventre, des brûlures d'estomac suivant le choix des composés pharmaceutiquement actifs. Cette liste n'est pas exhaustive.

Dans le traitement contre la douleur et la fièvre, on utilise préférentiellement des composés pharmaceutiquement actifs choisis parmi le paracétamol, l'acide acétylsalicilique ou l'ibuprofène.

Dans le traitement contre les nausées, vomissements et maux de voyage, on utilise préférentiellement la dompéridone.

Dans le traitement contre les diarrhées, on utilise préférentiellement la lopéramide.

Dans le traitement contre la toux, on utilise préférentiellement des composés pharmaceutiquement actifs choisis parmi la dextrométhorphane, la codéine, la carbocystéine, la N-acétylcystéine, la guaifénésine.

Dans le traitement contre les maux de gorge, on utilise préférentiellement la tyrothricine, le chlorure de cétylpyridinum, l'hexétidine.

Dans le traitement contre les maux de ventre, on utilise préférentiellement des extraits de plantes.

La composition selon l'invention permet l'intégration et donc la bonne conservation des composés pharmaceutiquement actifs.

Ces compositions sont susceptibles d'être stockées et conservées pendant un temps relativement long, par exemple de plusieurs mois environ, sans altération de leurs qualités. Les compositions réalisées selon l'invention sont dosées de manière classique pour les composés pharmaceutiquement actifs considérés.

## Revendications

1. Composition orosoluble, comprenant au moins un composé pharmaceutiquement actif présent, dans ladite composition, à une concentration supérieure ou égale à 10% en poids du poids total de la composition, **caractérisée en ce qu'**elle se présente sous la forme de granules comprenant:
un noyau obtenu par granulation en présence d'une phase solide poudreuse cristallisée et d'une phase liquide; et
plusieurs enrobages successifs obtenus par pulvérisation d'une phase liquide et saupoudrage d'une phase solide poudreuse cristallisée,
le composé pharmaceutiquement actif étant présent dans ledit noyau et/ou dans les enrobages successifs.

2. Composition selon la revendication 1, **caractérisée en ce que** la concentration en composé pharmaceutiquement actif est comprise entre 20% et 60% en poids du poids total de la composition.

3. Composition selon l'une des revendications 1 ou 2, **caractérisée en ce que** le composé pharmaceutiquement actif est présent dans les enrobages successifs et **en ce que** la concentration en composé pharmaceutiquement actif est homogène dans lesdits différents enrobages.

4. Composition selon la revendication 3, **caractérisée en ce que** le composé pharmaceutiquement actif est présent dans le noyau et **en ce que** la concentration en composé pharmaceutiquement actif est homogène dans le noyau et dans les enrobages successifs, soit du centre à la périphérie de la composition.

5. Composition selon l'une des revendications 1 ou 2, **caractérisée en ce que** la phase solide et la phase liquide pulvérisées dans l'étape d'enrobage sont différentes de celles utilisées dans l'étape de granulation, et/ou sont différentes d'une étape d'enrobage à une autre.

6. Composition selon l'une des revendications précédentes, **caractérisée en ce que** le noyau comporte une gomme.

7. Composition selon l'une des revendications précédentes, **caractérisée en ce que** le composé pharmaceutiquement actif est présent dans les enrobages successifs et **en ce que** la répartition du composé actif est sensiblement constante dans chacun des enrobages.

8. Composition selon l'une des revendications précédentes, **caractérisée en ce que** le composé pharmaceutiquement actif est choisi parmi le paracétamol, l'acide acétylsalicilique, l'ibuprofène, la dompéridone, la lopéramide le dextrométhorphane, la codéine, la carbocystéine, l'acétylcystéine, la N-acétylcystéine, l'acide hyaluronique, la guaifénésine, la tyrothricine, le chlorure de cétylpyridinum, l'hexétidine, pris seul ou en combinaison.

9. Procédé de fabrication d'une composition selon l'une des revendications 1 à 8, **caractérisé en ce qu'**il comprend les étapes suivantes de :
granulation en présence d'une phase solide poudreuse cristallisée et d'une phase liquide comprenant notamment une gomme ;
plusieurs enrobages successifs par pulvérisation d'une phase liquide et saupoudrage d'une phase solide poudreuse cristallisée, au moins un composé pharmaceutiquement actif étant présent dans la phase solide poudreuse et/ou dans la phase liquide, à l'étape de granulation ou d'enrobage ;
séchage à l'issue de chaque étape d'enrobage des granules enrobés obtenus ; et
tamisage en vue de l'obtention de granules calibrés.

10. Procédé selon la revendication 9, **caractérisé en ce que** le composé pharmaceutiquement actif est incorporé sous forme de poudre dans la phase solide poudreuse et/ou sous forme d'extrait liquide dans la phase liquide à l'étape de granulation et/ou d'enrobage.

11. Procédé selon l'une des revendications 9 ou 10, **caractérisé en ce qu'**il comprend une étape supplémentaire de réalisation d'une coque externe par pulvérisation d'une phase liquide comprenant un composé aromatique sur le granule préalablement formé.

12. Composition selon l'une des revendications 1 à 8 pour son utilisation comme médicament.

13. Composition pour utilisation selon la revendication 12 pour le traitement de la douleur, la fièvre, des nausées, des vomissements, des maux de voyage, des diarrhées, de la toux, des maux de gorge, des maux de ventre, des brûlures d'estomac.

## Patentansprüche

1. Im Mund lösliche Zusammensetzung, umfassend mindestens eine pharmazeutisch aktive Verbindung, die in der Zusammensetzung in einer Konzentration von mehr als oder gleich wie 10 Gew% des Gesamtgewichts der Zusammensetzung vorhanden ist, **dadurch gekennzeichnet, dass** sie die Form von Granulat aufweist, umfassend:
einen Kern, der durch Granulierung in Anwesenheit einer kristallisierten pulverförmigen festen Phase und einer flüssigen Phase erhalten wird; und
mehrere aufeinander folgende Umhüllungen, die durch Zerstäubung einer flüssigen Phase und Bestäuben einer kristallisierten pulverförmigen festen Phase erhalten werden,
wobei die pharmazeutisch aktive Verbindung in dem Kern und/oder in den aufeinander folgenden Umhüllungen vorhanden ist.

2. Zusammensetzung nach Anspruch 1, **dadurch gekennzeichnet, dass** die Konzentration von pharmazeutisch aktiver Verbindung im Bereich zwischen 20 Gew% und 60 Gew% mit Bezug auf das Gesamtgewicht der Zusammensetzung liegt.

3. Zusammensetzung nach einem der Ansprüche 1 oder 2, **dadurch gekennzeichnet, dass** die pharmazeutisch aktive Verbindung in den aufeinander folgenden Umhüllungen vorhanden ist, und dadurch, dass die Konzentration von pharmazeutisch aktiver Verbindung in den verschiedenen Umhüllungen homogen ist.

4. Zusammensetzung nach Anspruch 3, **dadurch gekennzeichnet, dass** die pharmazeutisch aktive Verbindung im Kern vorhanden ist, und dadurch, dass die Konzentration von pharmazeutische aktiver Verbindung im Kern und in den aufeinander folgenden Umhüllungen homogen ist, d. h. vom Zentrum zur Peripherie der Zusammensetzung.

5. Zusammensetzung nach einem der Ansprüche 1 oder 2, **dadurch gekennzeichnet, dass** die zerstäubte feste Phase und flüssige Phase im Schritt des Umhüllens verschieden von denjenigen sind, die im Schritt des Granulierens verwendet werden, und/oder von einem Schritt des Umhüllens zu einem anderen verschieden sind.

6. Zusammensetzung nach einem der vorhergehenden Ansprüche, **dadurch gekennzeichnet, dass** der Kern einen Gummi umfasst.

7. Zusammensetzung nach einem der vorhergehenden Ansprüche, **dadurch gekennzeichnet, dass** die pharmazeutisch aktive Verbindung in den aufeinander folgenden Umhüllungen vorhanden ist, und dadurch, dass die Verteilung der aktiven Verbindung im Wesentlichen in jeder der Umhüllungen konstant ist.

8. Zusammensetzung nach einem der vorhergehenden Ansprüche, **dadurch gekennzeichnet, dass** die pharmazeutisch aktive Verbindung ausgewählt ist aus Paracetamol, Acetylsalicylsäure, Ibuprofen, Domperidon, Loperamid, Dextromethorphan, Codein, Carbocystein, Acetylcystein, N-Acetylcystein, Hyaluronsäure, Guaifenesin, Tyrothricin, Cetylpyridiniumchlorid, Hexetidin, alleine oder in Kombination.

9. Verfahren zur Herstellung einer Zusammensetzung nach einem der Ansprüche 1 bis 8, **dadurch gekennzeichnet, dass** es die folgenden Schritte umfasst:
Granulieren in Anwesenheit einer kristallisierten pulverförmigen festen Phase und einer flüssigen Phase, umfassend insbesondere einen Gummi;
wiederholtes aufeinander folgendes Umhüllen durch Zerstäuben einer flüssigen Phase und Bestäuben einer kristallisierten pulverförmigen festen Phase, wobei mindestens eine pharmazeutisch aktive Verbindung in der pulverförmigen festen Phase und/oder in der flüssigen Phase im Schritt des Granulierens oder des Umhüllens vorhanden ist;
Trocknen am Ausgang jedes Schritts des Umhüllens der erhaltenen umhüllten Granulate; und
Sieben zum Erhalt von kalibrierten Granulaten.

10. Verfahren nach Anspruch 9, **dadurch gekennzeichnet, dass** die pharmazeutisch aktive Verbindung in Form eines Pulvers in die pulverförmige feste Phase und/oder in Form eines flüssigen Extrakts in der flüssigen Phase im Schritt des Granulierens und/oder des Umhüllens eingeschlossen wird.

11. Verfahren nach einem der Ansprüche 9 oder 10, **dadurch gekennzeichnet, dass** es einen zusätzlichen Schritt des Durchführens einer externen Schale durch Zerstäuben einer flüssigen Phase umfasst, umfassend eine aromatische Verbindung auf dem zuvor geformten Granulat.

12. Zusammensetzung nach einem der Ansprüche 1 bis 8 für ihre Verwendung als Arzneimittel.

13. Zusammensetzung für die Verwendung nach Anspruch 12 für die Behandlung von Schmerz, Fieber, Übelkeit, Erbrechen, Reisekrankheit, Durchfall, Husten, Halsweh, Bauchweh, Sodbrennen.

## Claims

1. Orosoluble composition, comprising at least one pharmaceutically active compound present, in said composition, at a concentration greater than or equal to 10% by weight of the total weight of the composition, **characterised in that** it is in the form of granules comprising:
a core obtained via granulation in the presence of a crystallised powdered solid phase and a liquid phase; and
a plurality of successive coatings obtained via spraying of a liquid phase and dusting with a crystallised powdered solid phase,
the pharmaceutically active compound being present in said core and/or in the successive coatings.

2. Composition according to claim 1, **characterised in that** the concentration of pharmaceutically active compound is between 20% and 60% by weight of the total weight of the composition.

3. Composition according to one of claims 1 and 2, **characterised in that** the pharmaceutically active compound is present in the successive coatings and **in that** the concentration of pharmaceutically active compound is homogenous in said various coatings.

4. Composition according to claim 3, **characterised in that** the pharmaceutically active compound is present in the core and **in that** the concentration of pharmaceutically active compound is homogenous in the core and in the successive coatings, or from the centre to the edge of the composition.

5. Composition according to one of claims 1 and 2, **characterised in that** the solid phase and the liquid phase sprayed in the coating step are different than those used in the granulation step, and/or are different from one coating step to another.

6. Composition according to one of the previous claims, **characterised in that** the core comprises a gum.

7. Composition according to one of the previous claims, **characterised in that** the pharmaceutically active compound is present in the successive coatings and **in that** the distribution of the active compound is substantially constant in each of these coatings.

8. Composition according to one of the previous claims, **characterised in that** the pharmaceutically active compound is chosen from paracetamol, acetylsalicylic acid, ibuprofen, domperidone, loperamide, dextromethorphan, codeine, carbocisteine, acetylcysteine, N-acetylcysteine, hyaluronic acid, guaifenesin, tyrothricin, cetylpyridinium chloride, hexetidine, taken alone or in combination.

9. Method for manufacturing a composition according to one of claims 1 to 8, **characterised in that** it comprises the following steps of:
granulation in the presence of a crystallised powdered solid phase and a liquid phase comprising in particular a gum;
a plurality of successive coatings via spraying of a liquid phase and dusting with a crystallised powdered solid phase, at least one pharmaceutically active compound being present in the powdered solid phase and/or in the liquid phase, during the step of granulation or coating;
drying, after each coating step, of the coated granules obtained; and
screening in order to obtain calibrated granules.

10. Method according to claim 9, **characterised in that** the pharmaceutically active compound is incorporated in the form of a powder in the powdered solid phase and/or in the form of a liquid extract in the liquid phase during the step of granulation and/or coating.

11. Method according to one of claims 9 and 10, **characterised in that** it comprises an additional step of creating an outer shell via spraying of a liquid phase comprising an aromatic compound onto the previously formed granule.

12. Composition according to one of claims 1 to 8, for its use as a drug.

13. Composition for use according to claim 12, for the treatment of pain, fever, nausea, vomiting, travel sickness, diarrhoea, cough, sore throat, stomach ache, heartburn.
